# EUROPÄISCHE PATENTSCHRIFT

(11) **EP 1 572 641 B1**
(45) Veröffentlichungstag und Bekanntmachung des Hinweises auf die Patenterteilung: **07.03.2012**
(21) Anmeldenummer: 03782435.6
(22) Anmeldetag: 18.12.2003
(51) Int. Cl.: C07D 207/12, C07D 409/12, A61K 31/381, A61K 31/40, A61K 31/4025, A61P 1/06, A61P 13/06, C07D 333/00, C07D 207/00

(54) **Verfahren zur Herstellung des R,R (oder S,S) konfigurierten Glycopyrronium-Stereoisomers**
Method for production of theR,R (or S,S) configuration of glycopyrronium stereoisomers
Procédé de préparation de stéreoisomères de glycopyrronium à configuration R,R (ou S,S)

(30) Priorität: 18.12.2002 AT 18962002
(43) Veröffentlichungstag der Anmeldung: 14.09.2005
(73) Patentinhaber: MEDA Pharma GmbH & Co. KG, 61352 Bad Homburg (DE)
(72) Erfinder: NOE, Christian, R., A-1180 Wien (AT); WALTER, Martin, R., 65830 Kriftel (DE)
(86) Internationale Anmeldenummer: PCT/EP2003/014432
(87) Internationale Veröffentlichungsnummer: WO 2004/054971

(56) Entgegenhaltungen:
- WO-A-98/21183
- WANG, Z. ET AL.: "USE OF CYCLODEXTRINS AS CHIRAL SELECTOR FOR THE CHIRAL SEPARATION OF ANTICHOLINERGIC DRUGS SUCH AS ANISODAMINE AND GLYCOPYRRONIUM IN CAPILLARY ZONE ELECTROPHORESIS" JOURNAL OF HIGH RESOLUTION CHROMATOGRAPHY., Bd. 19, 1. Dezember 1996 (1996-12-01), Seiten 697-699, XP002055747 DE WILEY VCH, WEINHEIM.

## Beschreibung

Die vorliegende Erfindung betrifft ein Verfahren zur Anreicherung der R,R- oder S,S-konfigurierten Glycopyrronium-Isomere und deren Thienyl-Analoga mit R,S- oder S,R-Konfiguration.

Muskarinrezeptoren blockierende Substanzen (Antimuskarinika) werden weltweit in gro-βem Umfang bei zahlreichen Krankheitsbildern eingesetzt (Goodman & Gilman's The Pharmacological Basis of Therapeutics, Ninth Edition, McGraw Hill, 1996; Mutschier Arzneimittelwirkungen, 8. Auflage, Wissenschaftliche Verlagsgesellschaft Stuttgart, 2001), wie z. B. zur Behandlung von chronisch obstruktiven Atemwegserkrankungen, Blasenentleerungsstörungen, Nieren- und Gallensteinkoliken sowie Irritationen der glatten Muskulatur des Magen-Darm-Kanals (u.a. bei Colon irritabile).

Diese Wirkungen werden von fünf verschiedenen Muskarinrezeptor-Subtypen vermittelt. Einzelne Ligand-Stereoisomere können unterschiedliche Affinitäten an diesen fünf Rezeptor-Subtypen aufweisen und damit verschiedene - erwünschte - Wirkungen gegenüber anderen - unerwünschten - bevorzugt hervorrufen. Selektive Substanzen sind nicht selektiven Wirkstoffen wegen derer geringeren Nebenwirkungen vorzuziehen, was in einigen Fällen durch den Einsatz einzelner reiner Stereoisomere erreicht werden kann.

Das Glykopyrronium-Isomere mit der R,R-Konfiguration weist besonders günstige pharmakologische Eigenschaften auf (WO9821183). Seine Herstellung ist beschrieben (WO9821183). Glykopyrroniumbromid bzw. davon abgeleitete Verbindungen beinhalten zwei stereogene Zentren, woraus sich die Existenz von jeweils vier Stereoisomeren ergibt.

Gegenstand der vorliegenden Erfindung ist ein Verfahren zur Herstellung von 3R,2'R-konfigurierten Glykopyrroniumsalzen bzw. von mit Glykopyrrominumbromid verwandten Verbindungen mit entsprechender räumlicher Anordnung (z.B. im Fall von Thienyl aufgrund der Cahn, Ingold, Prelog-Regel = 3R,2'S-Anordnung), dadurch gekennzeichnet, dass man aus dem enantiomerenreinen Diastereomerengemisch aus 3R,2'R- und 3R,2'S- oder aus 3R,2'R- und 3S,2'R-Isomer (bzw. im Fall der Thienyl-Analoga aus dem enantiomerenreinen Diastereomerengemisch aus 3R,2'S- und 3R,2'R- oder aus 3R, 2'S- und 3S,2'S-Isomer) mittels Verwendung eines geeigneten Lösungsmittel während der Quarternisierung und/oder mittels Umkristallisieren der quartären Salze das gewünschte Stereoisomer isoliert.

Das Verfahren ist grundsätzlich in gleicher Weise geeignet, die Antipoden (3S,2'S bei der Phenyl-Verbindung, bzw 3S,2'R beim Thienyl-Analogon) herzustellen, wenn man die Antipoden einsetzt (3S,2'S + 3S,2'R oder 3S,2'S + 3R,2'S bei der Phenyl-Verbindung, bzw 3S,2'R + 3S,2'S oder 3S,2'R + 3R,2'R beim Thienyl-Analogon).

Gegenstand der vorliegenden Erfindung ist demgemäß ein Verfahren
a) zur Isolierung des 3R,2'R-Stereoisomers von Glycopyrroniumbromid oder -iodid (Formel II: A = Br oder I), 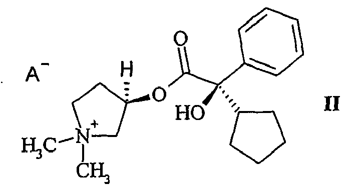 aus dem Diastereomerengemisch bestehend aus dem 3R,2'R- und 3R,2'S-Isomer (Formel III) 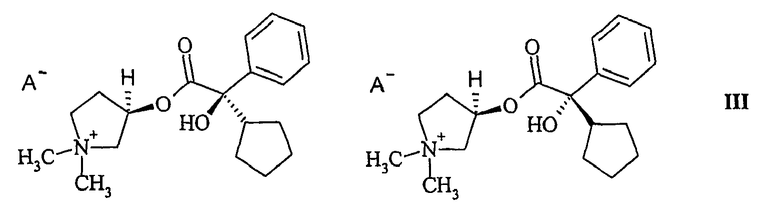 oder aus dem Diastereomerengemisch bestehend aus dem 3R,2'R- und 3S,2'R-Isomer (Formel IIIb) 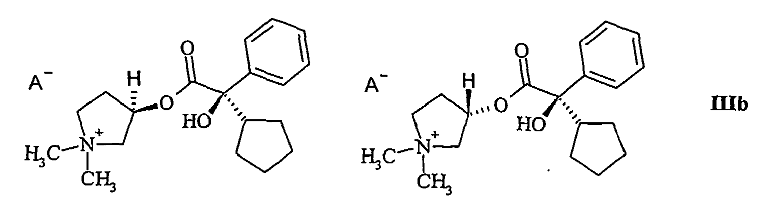 oder
b) zur Isolierung des 3S,2'S-Isomers (FormeIIV,_A = Br oder I) 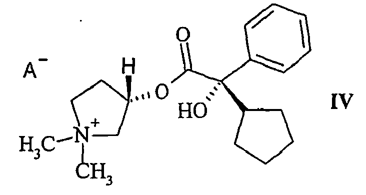 aus dem Diastereomerengemisch bestehend aus dem 3S,2'R- und 3S,2'S-Isomer (Formel V) 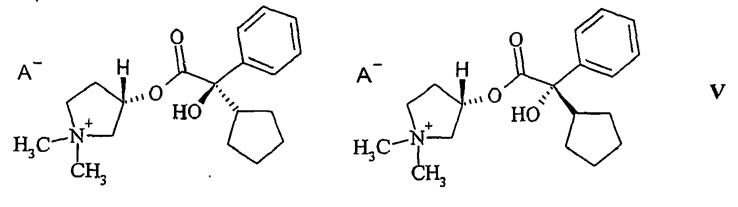 oder aus dem Diastereomerengemisch bestehend aus dem 3R,2'S- und 3S,2'S-Isomer (Formel Vb) 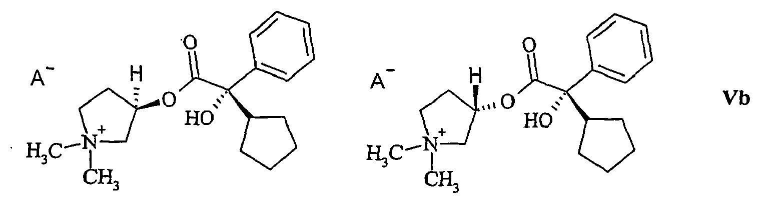 oder
c) zur Isolierung des 3R,2'S-Stereoisomers des Thienyl-Analogon von Glycopyrronium (Formel VI: A = Br oder I), 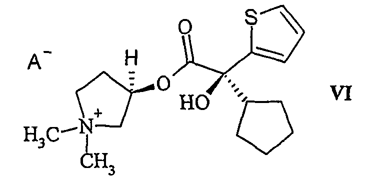 aus dem Diastereomerengemisch bestehend aus dem 3R,2'S- und 3R,2`R-Isomer (Formel VII) 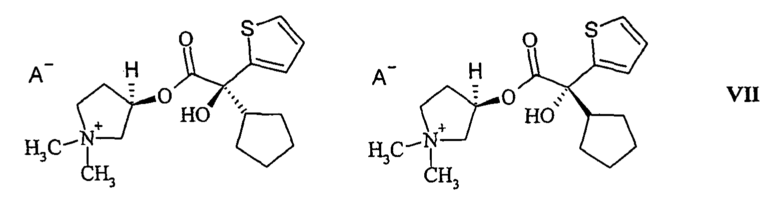 oder aus dem Diastereomerengemisch bestehend aus dem 3R,2'S- und 3S,2'S-Isomer (Formel VIIb) 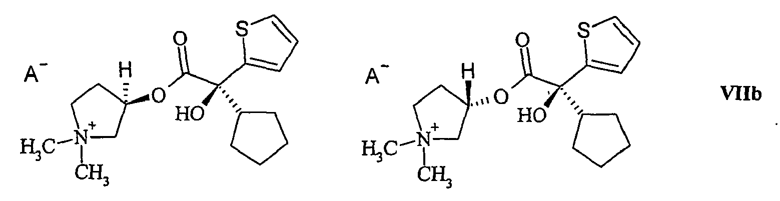 oder
d) zur Isolierung des 3S,2'R-Isomers (Formel VIII, A = Br oder I) 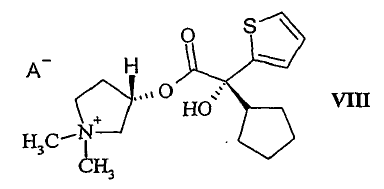 aus dem Diastereomerengemisch bestehend aus dem 3S,2'S- und 3S,2'R-Isomer (Formel IX) 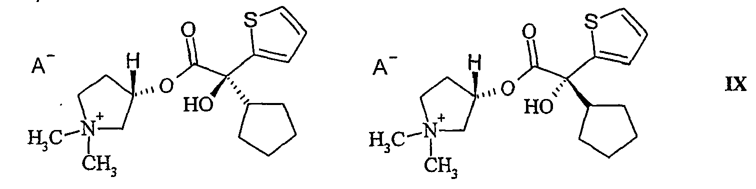 oder aus dem Diastereomerengemisch bestehend aus dem 3S,2'R- und 3R,2'R-Isomer (Formel IXb) 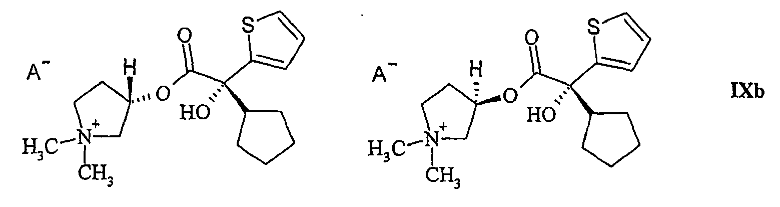
das dadurch gekennzeichnet ist,
dass man bei der Quarternisierung zu den oben genannten Diastereomerengemischen ein geeignetes Lösungsmittel verwendet, um das jeweils zu isolierende Stereoisomer angereichert als Niederschlag zu erhalten
und/oder
dass man die oben beschriebenen Diastereomerengemische der quartären Verbindungen in einem geeigneten Lösungsmittel oder -gemisch umkristallisiert und dabei das jeweils gewünschte Isomer angereichert erhält.

Bei einer der Ausführungsformen der Erfindung werden bei dem Verfahren Lösungsmittel oder Lösungsmittelgemische mit einem Wassergehalt verwendet, der dazu führt, dass nur das gewünschte Diastereomer kristallin anfällt, während das andere Diastereomer in Lösung bleibt oder als Öl anfällt.

Führt man die Quartemisierungen der Diastereomerengemische der zu Grunde liegenden tertiären Basen zu den genannten Diastereomerengemischen der Ammoniumsalze in einem geeigneten Lösungsmittel mit ausreichendem Wassergehalt durch, erhält man die erwünschten Stereoisomere im Niederschlag angereichert, während das jeweils andere Diastereomer in Lösung verbleibt.

Zur Umsetzung des erfindungsgemäßen Verfahrens kann zur Quarternisierung ein Lösungsmittel verwendet werden, in dem sich die tertiäre Base sowie die entstehenden quartären Salze lösen und der Zusatz eines weiteren Lösungsmittel die Kristallisation des erwünschten Isomers bewirkt.

Gemäß einem weiteren Merkmal des erfindungsgemäßen Verfahrens wird zur Quarternisierung ein Lösungsmittel verwendet, in dem sich beide Diastereomere der entstehenden quartären Salze schlecht lösen und der Zusatz eines weiteren Lösungsmittels dazu führt, dass das unerwünschte Isomer in Lösung geht oder als Öl anfällt.

Als geeignete Lösungsmittel für die Quarternisierung kann man beispielsweise verzweigte und unverzweigte Alkohole mit niederem Molekulargewicht, wie Methanol, Ethanol, Isopropanol, 1-Propanol, tert-Butanol, Isobutanol, n-Butanol, sowie Aceton, Butanon, Acetonitril und dergleichen, jeweils rein oder als Gemische mit anderen Lösungsmitteln, nennen.

In der Praxis wurde bei dem erfindungsgemäßen Verfahren zur Quarternisierung als Lösungsmittel bevorzugt Aceton, vorzugsweise mit einem Wassergehalt von etwa 0,5 - 2 % (bei einer Konzentration von etwa 5 - 20% Base in Lösungsmittel), noch bevorzugter mit einem Wassergehalt von etwa 1 % verwendet.

Gemäß einem weiteren Merkmal des erfindungsgemäßen Verfahrens wird zum Umkristallisieren ein Lösungsmittel verwendet, in dem sich das Diastereomerengemisch gut löst und ein zweites, die Kristallisation bewirkendes Lösungsmittel zugesetzt, um die Kristallisation herbeizuführen.

Bevorzugt werden/wird zum Lösen Methanol und/oder Ethanol verwendet und die Kristallisation mit Ethylacetat und/oder tert-Butylmethylether herbeigeführt.

Gemäß einem weiteren Merkmal wird bei dem erfindungsgemäßen Verfahren zum Umkristallisieren das Diastereomerengemisch in einem erhitzten Lösungsmittel gelöst und die Kristallisation durch Abkühlung erreicht.

Bevorzugt wird bei dem erfindungsgemäßen Verfahren zum Umkristallisieren das Diastereomerengemisch in der Siedehitze in 2-Propanol oder Ethanol gelöst und die Kristallisation bei Abkühlung auf Raumtemperatur oder darunter erreicht.

Bevorzugt wurde bei dem erfindungsgemäßen Verfahren zum Umkristallisieren das Diastereomerengemisch in der Siedehitze in 2-Propanol, welches einen Wassergehalt von etwa 0,2 - 3 % (bei einer Konzentration von etwa 5 - 30 % quartäres Ammoniumsalz in Lösungsmittel), noch bevorzugter etwa 0,5 % aufweist, gelöst und die Kristallisation durch Abkühlung erreicht.

Als geeignete Lösungsmittel für das Umkristallisieren kann man beispielsweise verzweigte und unverzweigte Alkohole mit niederem Molekulargewicht, wie Methanol, Ethanol, Isopropanol, 1-Propanol, tert-Butanol, Isobutanol, n-Butanol, sowie Aceton, Butanon, Acetonitril und dergleichen, jeweils rein oder als Gemische mit anderen Lösungsmitteln, nennen.

Gemäß einem weiteren Merkmal der vorliegenden Erfindung wird das erfindungsgemäße Verfahren bevorzugt zur Anreicherung des 3R,2'R-Isomers von Glycopyrroniumbromid herangezogen.

Sowohl bei der Quarternisierung der zu Grunde liegenden tertiären Basen zu den genannten Diastereomerengemische der Ammoniumsalze als auch beim Umkristallisieren der quartären Salze wird durch Nachwaschen der Niederschläge des erwünschten Stereoisomers an dessen Oberfläche anhaftendes unerwünschtes Isomer entfernt. Dieses Nachwaschen kann auch noch nach Isolierung des Rohproduktes durchgeführt werden, indem man das Rohprodukt in einem Lösungsmittel, in welchem es nur teilweise gelöst wird (bevorzugt Aceton mit einem Wassergehalt von 1%), digeriert und anschließend den vom unerwünschten Diastereomer gereinigten Niederschlag absaugt.

Das erfindungsgemäße Verfahren kann auch in Kombination mit anderen Verfahren zur Steigerung der Steroisomerenreinheit vorteilhaft genutzt werden:
Sollten z. B. die zur Quarternisierung eingesetzten Basen oder die quartären Ammoniumsalze bereits Diastereomeren-angereichert vorliegen, so kann diese Anreicherung mit dem erfindungsgemäßen Verfahren optimiert werden.

Andererseits kann das erfindungsgemäße Verfahren im Sinne einer Vorreinigung bereits einen hohen Stereoisomerenüberschuss bewirken, der bei Anwendung eines darauf folgenden Verfahrens zur weiteren Steigerung der Stereoisomerenreinheit vorteilhaft ist.

Das beanspruchte Verfahren ist geeignet, aus dem bei Verwendung des 3R-konfigurierten enantiomerenreinen Aminoalkohols und der razemischen Säure (oder einem entsprechenden Ester oder einem aktivierten Säurederivat) entstehende 3R,2'R- / 3R,2'S-Diastereomerengemisch des tertiären Glycopyrrolats bzw. bei Verwendung von 3S-konfiguriertem Aminoalkohol das entstehende Gemisch aus 3S,2'R- und 3S,2'S-Isomeren mittels Verwendung eines geeigneten Lösungsmittels während der Quarternisierung und/oder durch Umkristallisieren der quartären Ammoniumsalze das Isomer anzureichern, bei welchem die asymmetrischen Zentren der Aminoalkoholkomponente und der Säurekomponente die selbe Bezeichnung der Absolutkonfiguration tragen (also 3R,2'R und 3S,2'S; Formel Ia). Durch Wiederholen der Kristallisationprozedur lässt sich die Stereoisomerenreinheiten weiter perfektionieren.

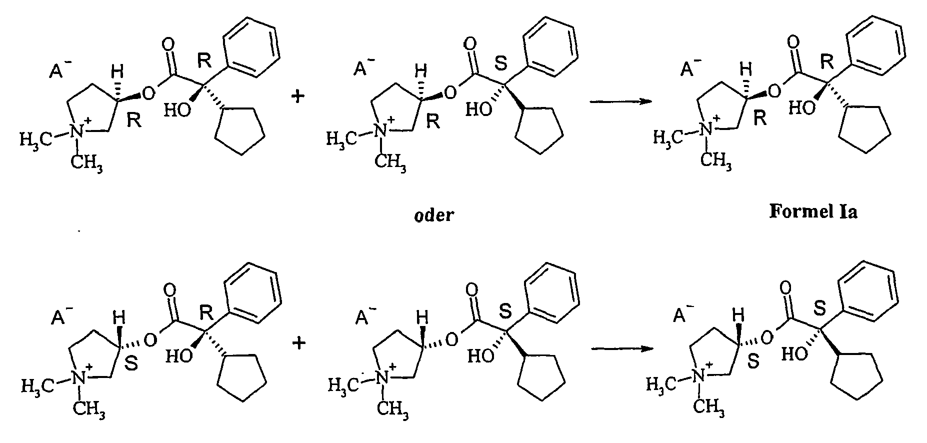

Im Falle des 2-Thienyl-Analogon erhält man aus dem Diastereomerengemisch aus 3R,2'S- und 3R,2'R-Isomer bzw. aus dem 3S,2'R-/3S,2'S-Diastereomerengemisch auf Grund der höheren Priorität des Thienylrestes die Stereoisomere, welche die entgegengesetzte Bezeichnung der Konfiguration tragen (also 3R,2'S und 3S,2'R; Formel Ib), aber die selbe räumliche Anordnung wie bei der Phenyl-Verbindung aufweisen.

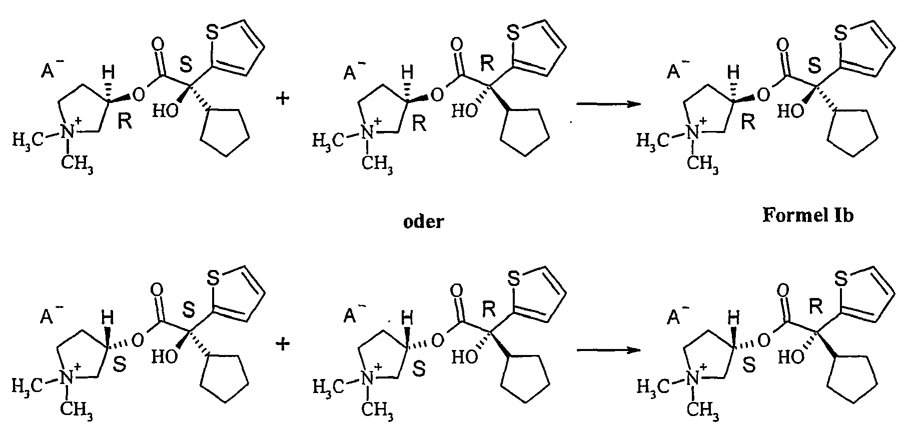

Alternativ dazu kann man auch von einer enantiomerenreinen Säurekomponente (oder einem entsprechenden Ester oder einem aktivierten Säurederivat) und einem razemischen Aminoalkohol ausgehen und aus dem resultierenden basischen Diastereomerengemisch mittels Verwendung eines geeigneten Lösungsmittel während der Quarternisierung und/oder mittels Umkristallisieren der quartären Salze das gewünschte Stereoisomer isolieren.

Im Falle der 2R-konfigurierten Säurekomponente erhält man dabei aus dem entstehenden 3R,2'R- / 3S,2'R- Diastereomerengemisch des tertiären Glycopyrrolats bzw. bei Verwendung von 2S-konfigurierter Säurekomponente aus dem entstehenden Gemisch aus 3R,2'S- und 3S,2'S-Isomeren mittels Verwendung eines geeigneten Lösungsmittel während der Quarternisierung und/oder durch Umkristallisieren der quartären Ammoniumsalze das Isomer, bei welchem die asymmetrischen Zentren der Aminoalkoholkomponente und der Säurekomponente die selbe Bezeichnung der Absolutkonfiguration tragen (also 3R,2'R bzw. 3S,2'S; Formel Ic).

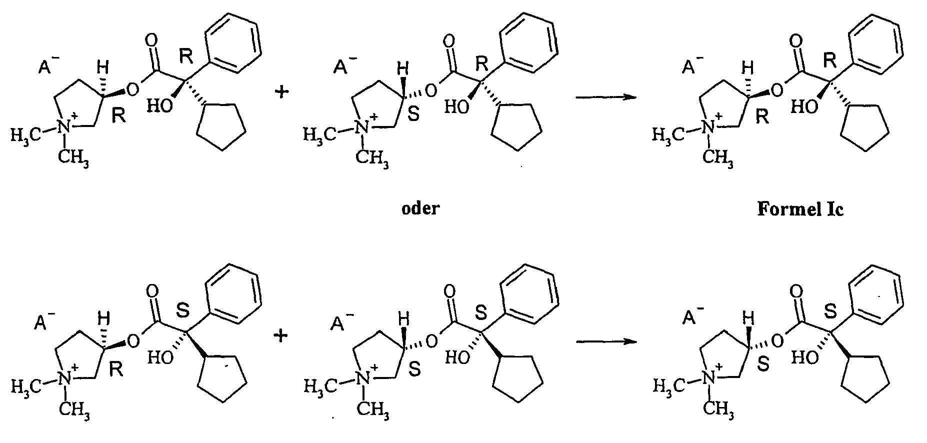

Im Falle des 2-Thienyl-Analogon erhält man aus dem Diastereomerengemisch aus 3R,2'S- und 3S.2'S-Isomer bzw. aus dem 3R,2'R-/3S,2'R-Diastereomerengemisch auf Grund der höheren Priorität des Thienylrestes die Stereoisomere, welche die entgegengesetzte Bezeichnung der Konfiguration tragen (also 3R,2'S und 3S,2'R; Formel Id), aber die selbe räumliche Anordnung wie bei der Phenyl-Verbindung aufweisen.

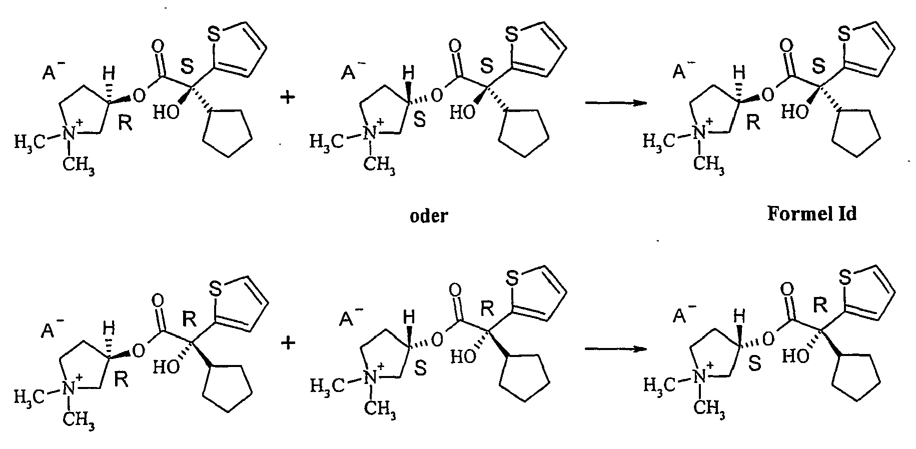

Kristallisiert man im Falle des Glycopyrroniumbromids dagegen ein Gemisch aller vier Isomere des quartären Ammoniumsalzes um, so erhält man als Feststoff eine Mischung aus 3R,2'S- und 3S,2'R-Isomer, d.h. das Isomer mit den vorteilhaftesten pharmakologischen Eigenschaften geht bei dieser Methode verloren.

Zur Isolierung der genannten Stereoisomere aus den Diastereomerengemischen quartärer Ammoniumsalze ist der Einsatz verschiedener Kristallisationsmethoden möglich. Als Solventien zur Umkristallisation sind allgemein alle Lösungsmittel und Lösungsmittelgemische geeignet, in denen das Diastereomerengemisch - gegebenenfalls durch Erhitzen - in Lösung gebracht und durch geeignete Maßnahmen Kristallisation hervorgerufen werden kann, wobei im Feststoff das gewünschte Isomer angereichert anfällt.

Als geeignete Maßnahmen, um in der Lösung des Diastereomerengemisches Kristallisation zu initiieren, seien genannt: Abkühlen der Lösung, Zusatz von weiteren Lösungsmitteln, in denen das gewünschte Isomer eine geringere Löslichkeit als im ursprünglichen Lösungsmittel aufweist, Einengen des Lösungsmittelvolumens, Entfernen einer Komponente des Lösungsmittelgemisches, in der das gewünschte Isomer eine höhere Löslichkeit aufweist, als im verbleibenden Lösungsmittelgemisch.

Wie festgestellt wurde, zeigt das unerwünschte Isomer ein stark hygroskopisches Verhalten, während dies bei dem gewünschten Isomer nicht zu beobachten ist. Bei einer der Ausführungsformen des beanspruchten Verfahren, bei welchem ein Lösungsmittel mit ausreichendem Wassergehalt verwendet wird bzw. einem wasserfreien Lösungsmittel Wasser zugesetzt wird, führt diese Eigenart dazu, dass ausschließlich das gewünschte Diastereomer kristallin anfällt, während das andere Diastereomer in Lösung bleibt oder ein abtrennbares Öl bildet.

Bevorzugt wird die Verwendung von flüchtige Lösungsmittel, die aus dem gewonnenen Feststoff leicht zu entfernen sind.

Besonders bevorzugt sind Lösungsmittel, aus denen ausschließlich das gewünschte Stereoisomer als Feststoff kristallisiert.

Das hier beanspruchte Verfahren erlaubt es, mit geringem technischen Aufwand kostengünstig das gewünschte Stereoisomer in sehr hoher Reinheit zu erhalten.

Der Verbrauch an Edukten auf Grund der Isomerentrennung nach dem letzten Reaktionsschritt ist relativ hoch gegenüber anderen denkbaren Verfahren, bei denen die Trennung von Isomeren bei einem früheren Abschnitt des Syntheseweges erfolgt. Dieser vermeintliche Nachteil wird jedoch durch die einfache Durchführbarkeit des beanspruchten Verfahrens überkompensiert.

Insbesondere die Tatsache, dass bei diesem Verfahren das gewünschte Isomer gegenüber dem unerwünschten Diastereomer eine drastisch erhöhte Tendenz zum Kristallisieren aufweist, macht den besonderen Vorteil dieses Verfahrens aus und verdient besondere Beachtung.

Die enantiomerenreine Aminoalkoholkomponente kann nach Techniken hergestellt werden, die in der Literatur beschrieben sind (Razematspaltung und asymmetrische Synthese: J. Med. Chem. 34 (1991) 1314-1328). Selbiges gilt auch für die Säurekomponente: (Razematspaltung: Bioorg. Med. Chem. 7 (1999) 2555-2567; asymmetrische Synthese: Bioorg. Med. Chem. Lett. 9 (1999) 2037-2038). Somit ist die Durchführbarkeit des Verfahrens gegeben.

Im Hinblick auf die technische Verwertbarkeit zeigt die erfindungsgemäße Methode deutliche Vorteile gegenüber anderen denkbaren Verfahren zur Herstellung des genannten Stereoisomers aus dem jeweiligen Diastereomerengemisch. So sind bei dem erfindungsgemäßen Verfahren keine zusätzlichen, zum Teil sehr teuren und eventuell in größeren Mengen überhaupt nicht zugänglichen, chiralen Auxiliare nötig. Der Einsatz einer einzigen enantiomerenreinen Substanz, die ohnehin Baustein des Produktes ist, reicht aus. Damit ist die Herstellung des reinen Isomers kaum aufwändiger, als die Produktion des Gemisches aus allen vier Isomeren, oder gar des sich derzeit im Handel befindlichen Isomerengemisches aus 3R,2'S- und 3S,2'R-Glycopyrroniumbromid. Im letztgenannten Fall ist das therapeutisch sinnvollste Isomer nicht einmal im Produkt enthalten.

Außerdem vermeidet das erfindungsgemäße Verfahren zusätzliche Arbeitsschritte, die bei Razematspaltungen nötig sind, wie Kristallisieren mit chiralen Basen oder Säuren, mehrfaches Umkristallisieren der entstehenden Salze, Freisetzen aus den genannten Salzen und im Falle der Säurekomponente eine vorhergehende Verseifung des Esters und eine erneute Methylesterbildung.

Die nachfolgenden Beispiele dienen der Beschreibung der Erfindung.

### Beispiel 1

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

### Umesterung

In einer trockenen Reaktionsapparatur werden 0,17 mol (3R)-1-Methyl-3-pyrrolidinol und 0,17 mol razemischer 2-Cyclopentyl-2-hydroxy-2-phenylessigsäuremethylester in 800 ml n-Heptan abs. vorgelegt. Anschließend werden 400 ml Heptan zum Entfernen aller Feuchtigkeitsspuren destillativ entfernt und durch den Wasserabscheider abgelassen. Nach dem Abkühlen werden 0,9 g NaOMe (10 mol%) zugesetzt und wiederum zum Sieden erhitzt. Die übergehende Menge Lösungsmittel wird über 5-6 h fortlaufend mittels Tropftrichter ersetzt. Nach wässriger Aufarbeitung des Reaktionsgemisches und Extraktion mit Ether wird die organische Phase über Na₂SO₄ / K₂CO₃ 2:1 getrocknet. Entfernen von Trocken- und Lösungsmittel liefert die freie Base mit 82% Ausbeute.

Quarternisierung: Die freie Base wird durch Zugabe von 3 eq Methylbromid, gelöst in tert-Butylmethylether, quarterniert und das kristallin anfallende Produkt mit 93% Ausbeute abgesaugt. Die entstandene Diastereomerenmischung aus (3R,2'S)- und (3R,2'R)-[(2-Cyclopentyl-2-hydroxy-2-phenylacetyl)oxy]-1,1-dimethylpyrrolidiniumbromid liegt im Verhältnis von etwa eins zu eins vor.

### Diastereomerentrennung durch Umkristallisation:

Das Rohprodukt wird in 250 ml Methanol gelöst, mit 300 ml Ethylacetat verdünnt und mit 450 ml Diethylether versetzt. Nach zwei Tagen werden die entstandenen Kristalle abgesaugt und ein zweites Mal umkristallisiert. Man erhält in 50%-iger Ausbeute das (3R,2'R)-Isomer.

### Analytische Daten:

### Aussehen: farblose Kristalle

¹H-NMR (D₂O) (300 MHz): δ = 7.55-7.52 (m, 2H, Phenyl); 7.37-7.24 (m, 3H, Phenyl); 5.42-5.34 (m, 1H); 4.67 (s, H₂O); 3.65 (dd, 1H); 3.61-3.43 (m, 2H); 3.40 (dd, 1H,); 3.13-2.98 (m, 1H, Cyclopentyl-Methin); 3.04 (s, 3H, NCH₃, (3R,2'R)); 2.75 (s, 3H, NCH₃, (3R,2'R)); 2.71-2.55 (m, 1H); 2.30-2.17 (m, 1H (3R,2'R)); 1.65-1.43 (m, 7H, Cyclopentyl); 1.19-1.05 (m, 1H)

Die dazu diastereomeren Verbindungen unterscheiden sich im Wesentlichen durch die Verschiebungen folgender Signale: 3.08 (s, NCH₃); 2.91 (s, NCH₃); 2.11-1.92 (m)

Aus dem Vergleich der Integrale der Signale bei 2.75 (s, 3H, NCH₃, (3R,2'R)) und 2.91 (s, NCH₃ (Diastereomere)) ergibt sich für das R,R-Glycopyrroniumbromid ein Diastereomerenüberschuss von über 98% de. Die Enantiomerenreinheit ergibt sich aus der Verwendung von enantiomerenreinem 3R-N-Methylpyrrolidinol.

¹³C-NMR (D₂O) (50 MHz): δ = 177.1 (s, 1'-COO); 143.2 (s, Phenyl); 131.4 (d, Phenyl); 131.0 (d, Phenyl); 128.8 (d, Phenyl); 83.2 (s); 76.6 (d); 72.9 (t); 67.6 (t); 56.3 (q, NCH₃); 55.6 (q, NCH₃); 47.5 (d, Cyclopentyl-Methin); 32.4 (t); 29.2/28.8/28.7/28.4 (t, Cyclopentyl-Methylen)
Summenformel / Masse des Kations: (C₁₉H₂₈NO₃)⁺ (Br)⁻ / 318.44;
(ESI+)-Massenspektrum: 318.2 = M⁺
Elementaranalyse: Ber.: C 57,29 H 7,09 N 3,52; Gef.: C 57,41 H 7,00 N 3,54

Die Bestimmung der Absolutkonfiguration erfolgte mittels Röntgenstrukturanalyse.

### Beispiel 2

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

Umesterung und Quarternisierung werden wie unter Beispiel 1 beschrieben durchgeführt.

Die Diastereomerentrennung erfolgt mittels Umkristallisieren des Diastereomerengemisches in Isopropanol. Dazu wird das Rohprodukt in der Siedehitze in der 8-fachen Gewichtsmenge Isopropanol gelöst. Man lässt über Nacht bei Raumtemperatur stehen und saugt die entstandenen Kristalle ab. Diese Prozedur wird noch einmal wiederholt. Das so gewonnene (3R,2'R)-Glycopyrroniumbromid führt zu den selben analytischen Daten, wie unter Beispiel 1 beschrieben.

### Beispiel 3

### Herstellung von (3R,2'R)-[(2-Cyclopentyl-2-hydroxy-2-phenylacetyl)oxy]-1,1-dimethylpyrrolidiniumiodid

### (Formel la: A⁻ = I⁻)

Umesterung, Quarternisierung (mit Methyliodid) und Diastereomerentrennung werden wie unter Beispiel 1 beschrieben durchgeführt.

Das so gewonnene (3R,2'R)-Glycopyrroniumiodid führt zu den selben NMR-spektroskopischen Daten, wie unter Beispiel 1 beschrieben.

### Beispiel 4

### Herstellung von (3R,2'S)-3-[(2'-Cyclopentyl-2'-hydroxy-2'-(2"-thienyl)acetyl)oxy]-1,1-dimethylpyrrolidiniumbromid

### (Formel Ib: A⁻ = Br⁻)

Umesterung (mit (3R)-1-Methyl-3-pyrrolidinol und razemischem 2-Cyclopentyl-2-hydroxy-2-(2'-thienyl)essigsäuremethylester), Quarternisierung und Diastereomerentrennung werden wie unter Beispiel 1 beschrieben durchgeführt.

¹H-NMR (300 MHz, D₂O): δ = 7.33 (d, 1H, Thienyl); 7.13 (d, 1H, Thienyl); 6.96 (dd, 1H, Thienyl); 5.50-5.42 (m, 1H); 4.67 (s, H₂O); 3.78-3.62 (m, 2H); 3.58-3.47 (m, 2H); 3.08 (s, 3H, N-CH₃, (3R,2'S)); 3.01-2.83 (m, 4H, N-CH₃, (3R,2'S) und Cyclopentyl-Methin); 2.76-2.62 (m, 1 H); 2.37-2.22 (m, 1 H (3R,2'S)); 1.65-1.20 (m, 8H, Cyclopentyl-Methylen).

Die dazu diastereomeren Verbindungen (3R,2'R und 3S,2'S) unterscheiden sich im Wesentlichen durch die Verschiebungen folgender Signale: 3.11 (s, NCH₃); 3.04 (s, NCH₃); 2.15-2.03 (m).

Der Diastereomerenüberschuss wurde mittels kapillar-elektrophoretischer Methode auf über 98% de bestimmt. Die Enantiomerenreinheit ergibt sich aus der Verwendung von enantiomerenreinem 3R-N-Methylpyrrolidinol.

¹³C-NMR (50 MHz, D₂O): δ = 176.2 (s, 1'-COO); 147.7 (s, Thienyl); 130.1 (d, Thienyl); 128.8 (d, Thienyl); 128.4 (d, Thienyl); 82.2 (s); 76.9 (d); 72.9 (t); 67.7 (t); 56.4 (q, NCH₃); 55.8 (q, NCH₃); 49.4 (d, Cyclopentyl-Methin); 32.5 (t); 29.2/29.0/28.7/28.4 (t, Cyclopentyl-Methylen).
Summenformel / Masse des Kations: (C₁₇H₂₆NO₃S)⁺ (Br)⁻ / 324.47
(ESI+)-Massenspektrum : 324.4 = M⁺.

Die Bestimmung der Absolutkonfiguration erfolgte mittels Röntgenstrukturanalyse.

### Beispiel 5

### Herstellung von (3S,2'S)-[(2-Cyclopentyl-2-hydroxy-2-phenylacetyl)oxy]-1,1-dimethylpyrrolidiniumbromid

### (Formel la: A⁻ = Br⁻)

Umesterung (mit (3S)-1-Methyl-3-pyrrolidinol und razemischem 2-Cyclopentyl-2-hydroxy-2-phenylessigsäuremethylester), Quarternisierung und Diastereomerentrennung werden wie unter Beispiel 1 beschrieben durchgeführt.

Die NMR-spektroskopischen Daten entsprechen den in Beispiel 1 für die enantiomere Verbindung angegebenen Werten.

### Beispiel 6

### Herstellung von (3S,2'R)-3-[(2'-Cyclopentyl-2'-hydroxy-2'-(2"-thienyl)acetyl)oxy]-1,1-dimethylpyrrolidiniumbromid

### (Formel Ib: A⁻ = Br⁻)

Umesterung (mit (3S)-1-Methyl-3-pyrrolidinol und razemischem 2-Cyclopentyl-2-hydroxy-2-(2'-thienyl)essigsäuremethylester), Quarternisierung und Diastereomerentrennung werden wie unter Beispiel 1 beschrieben durchgeführt.

Die NMR-spektroskopischen Daten entsprechen den in Beispiel 4 für die enantiomere Verbindung angegebenen Werten.

### Beispiel 7

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

Umesterung (mit razemischem 1-Methyl-3-pyrrolidinol und (2R)-2-Cyclopentyl-2-hydroxy-2-phenylessigsäuremethylester) und Quarternisierung werden wie unter Beispiel 1 beschrieben durchgeführt.

Die Diastereomerentrennung erfolgt wie in Beispiel 2 beschrieben, mittels Umkristallisieren des Diastereomerengemisches in Isopropanol.

Die NMR-spektroskopischen Daten entsprechen den unter Beispiel 1 angegebenen Werten.

### Beispiel 8

### Herstellung von (3R,2'S)-3-[(2'-Cyclopentyl-2'-hydroxy-2'-(2"-thienyl)acetyl)oxy]-1,1-dimethylpyrrolidiniumbromid

### (Formel Ib: A⁻ = Br⁻)

Umesterung (mit razemischem 1-Methyl-3-pyrrolidinol und (2S)-2-Cyclopentyl-2-hydroxy-2-(2'-thienyl)essigsäuremethylester) und Quarternisierung werden wie unter Beispiel 1 beschrieben durchgeführt.

Die Diastereomerentrennung erfolgt wie in Beispiel 1 beschrieben, mittels Umkristallisieren des Diastereomerengemisches in einer Mischung aus Methanol, Ethylacetat und Diethylether.

Die NMR-spektroskopischen Daten entsprechen den unter Beispiel 4 angegebenen Werten.

### Beispiel 9

### Herstellung von (3S,2'S)-[(2-Cyclopentyl-2-hydroxy-2-phenylacetyl)oxy]-1,1-dimethylpyrrolidiniumbromid

### (Formel la: A⁻ = Br⁻)

Umesterung (mit razemischem 1-Methyl-3-pyrrolidinol und (2S)-2-Cyclopentyl-2-hydroxy-2-phenylessigsäuremethylester) und Quarternisierung werden wie unter Beispiel 1 beschrieben durchgeführt.

Die Diastereomerentrennung erfolgt wie in Beispiel 2 beschrieben, mittels Umkristallisieren des Diastereomerengemisches in Isopropanol.

Die NMR-spektroskopischen Daten entsprechen den in Beispiel 1 für die enantiomere Verbindung angegebenen Werten.

### Beispiel 10

### Herstellung von (3S,2'R)-3-[(2'-Cyclopentyl-2'-hydroxy-2'-(2"-thienyl)acetyl)oxy]-1,1-dimethylpyrrolidiniumbromid

### (Formel Ib: A⁻ = Br⁻)

Umesterung (mit razemischem 1-Methyl-3-pyrrolidinol und (2R)-2-Cyclopentyl-2-hydroxy-2-(2'-thienyl)essigsäuremethylester) und Quarternisierung werden wie unter Beispiel 1 beschrieben durchgeführt.

Die Diastereomerentrennung erfolgt wie in Beispiel 1 beschrieben, mittels Umkristallisieren des Diastereomerengemisches in einer Mischung aus Methanol, Ethylacetat und Diethylether.

Die NMR-spektroskopischen Daten entsprechen den in Beispiel 4 für die enantiomere Verbindung angegebenen Werten.

### Beispiel 11

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

Umesterung und Quarternisierung werden wie unter Beispiel 1 beschrieben durchgeführt, mit dem Unterschied, dass man die Methylierung in Isopropanol durchführt. Absaugen des entstehenden Niederschlages liefert ein Diastereomerenverhältnis von 98% 3R,2'R- und 2% 3R,2'S-Isomer.

Die weitere Diastereomerentrennung erfolgt wie in Beispiel 2 beschrieben. Da zur Umkristallisation bereits diastereomerenangereichertes Rohprodukt verwendet wird, erhält man eine höhere Stereoisomerenreinheit bei gleicher Anzahl der Kristallisationsschritte bzw. man benötigt zur gleichen Anreicherung eine geringere Zahl an Umkristallisationen.

Das so gewonnene (3R,2'R)-Glycopyrroniumbromid führt zu den selben analytischen Daten, wie unter Beispiel 1 beschrieben.

### Beispiel 12

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

Umesterung und Quarternisierung werden wie unter Beispiel 1 beschrieben durchgeführt, mit dem Unterschied, dass man die Methylierung in Aceton durchführt. Absaugen des entstehenden Niederschlages liefert ein Diastereomerenverhältnis von 90% 3R,2'Rund 10% 3R,2'S-Isomer.

Die weitere Diastereomerentrennung erfolgt wie in Beispiel 11 beschrieben.

Das so gewonnene (3R,2'R)-Glycopyrroniumbromid führt zu den selben analytischen Daten, wie unter Beispiel 1 beschrieben.

### Beispiel 13

### Herstellung von (3R,2'R)-[(2-Cyclopentyl-2-hydroxy-2-phenylacetyl)oxy]-1,1-dimethylpyrrolidinium bromid.

Die Umesterung wird wie unter Beispiel 1 beschrieben durchgeführt.

### Quarternisierung:

Die freie Base, gelöst in Aceton, welches einen Wassergehalt von 1 % aufweist, wird durch Zugabe von 1,3 eq Methylbromid quarterniert und das kristallin anfallende Produkt mit etwa 74% Ausbeute (bezogen auf erwünschtes Diastereomer) abgesaugt. Das Diastereomerenverhältnis beträgt 95 zu 5 oder besser.

Die weitere Diastereomerentrennung erfolgt wie in Beispiel 2 beschrieben. Da zur Umkristallisation bereits diastereomerenangereichertes Rohprodukt verwendet wird, erhält man eine höhere Stereoisomerenreinheit bei gleicher Anzahl der Kristallisationsschritte bzw. man benötigt zur gleichen Anreicherung eine geringere Zahl an Umkristallisationen.

Das so gewonnene (3R,2'R)-Glycopyrroniumbromid führt zu den selben analytischen Daten, wie unter Beispiel 1 beschrieben.

Bei ansonsten gleicher Vorgehensweise, aber unter Verwendung von Aceton mit einem Wassergehalt zwischen 0,5 und 2%, in dem soviel Base gelöst wird, dass sie bezüglich der Lösungsmittelmenge mit einer Konzentration zwischen 5 und 20 Gewichtsprozent vorliegt, erhält man bei der Quarternisierung hinsichtlich der Diastereomerenanreicherung vergleichbare Ergebnisse.

### Beispiel 14

### Herstellung von (3R,2'R)-[(2-Cyclopentyl-2-hydroxy-2-phenylacetyl)oxy]-1,1-dimethyl pyrrolidinium bromid.

Die Umesterung wird wie unter Beispiel 1 beschrieben durchgeführt, die Quarternisierung wie in Beispiel 13 beschrieben.

Die Diastereomerentrennung erfolgt mittels Umkristallisieren des Diastereomerengemisches in Isopropanol, welches einen Wassergehalt von 0,5% aufweist. Nach nur einmaliger Wiederholung der Umkristallisierung beträgt der Anteil an unerwünschtem Diastereomer weniger als 0,5%.

Das so gewonnene (3R,2'R)-Glycopyrroniumbromid führt zu den selben analytischen Daten, wie unter Beispiel 1 beschrieben.

Bei ansonsten gleicher Vorgehensweise, aber unter Verwendung von Isopropanol mit einem Wassergehalt zwischen 0,2% und 3%, von dem soviel zugesetzt wird, dass das Rohprodukt bezüglich der Lösungsmittelmenge mit einer Konzentration zwischen 5 und 30 Gewichtsprozent vorliegt, erhält man nach zweimaligem Umkristallisieren hinsichtlich der Diastereomerenanreicherung vergleichbare Ergebnisse.

### Beispiel 15

### Herstellung von (3R,2'R)-[(2-Cyclopentyl-2-hydroxy-2-phenylacetyl)oxy]-1,1-dimethylpyrrolidiniumbromid.

Die Umesterung wird ähnlich wie unter Beispiel 1 beschrieben durchgeführt. Quarternisierung:
6 kg der Glycopyrrolat-Base werden in 55 Liter Aceton, welches einen Wassergehalt von etwa 1 % aufweist, gelöst und die Mischung auf -5°C (± 5) abgekühlt. Man leitet 1,3 Äquivalente gasförmiges Brommethan langsam ein, so dass es in die gekühlte Lösung einkondensieren kann. Anschließend erwärmt man während 3 Stunden auf Raumtemperatur und lässt weitere 3 Stunden rühren. Der entstandene Niederschlag wird abfiltriert und zum Entfernen von Methylbromid aus dem Produkt mit 10 Liter Aceton nachgewaschen.

Durch Trocknen erhält man das Rohprodukt als kristallinen, weißen Niederschlag in etwa 70% Ausbeute (bezogen auf erwünschtes Diastereomer), worin weniger als 5 % des unerwünschten Diastereomers enthalten sind.

### Umkristallisation:

Zum Niederschlag gibt man 20 Liter Isopropanol zu, welches einen Wassergehalt von 0,5% Wasser aufweist und erhitzt diese Mischung unter Rückfluss. Wenn sich der Feststoff vollständig gelöst hat, filtriert man die Lösung, um von Schwebstoffen zu reinigen. Zur Kristallisation kühlt man die Lösung über einen Zeitraum von 3 Stunden auf Raumtemperatur ab. Nach weiteren 3 Stunden, kann man das ausgefallene Produkt absaugen, wobei mit 5 Liter Isopropanol nachgewaschen wird.

Eine einmalige Wiederholung des Umkristallisationsvorgangs reicht aus, um (3R,2'R)-Glycopyrroniumbromid in einer Qualität zu erhalten, welche die Anforderungen an einen Wirkstoff zur Herstellung von Arzneimittel übertrifft, d.h. es sind in Summe weniger als 0,5% der anderen drei Stereoisomere und weniger als 1 % Gesamtverunreinigungen enthalten.

### Beispiel 16

### Herstellung von (3R,2'R)-[(2-Cyclopentyl-2-hydroxy-2-phenylacetyl)oxy]-1,1-dimethylpyrrolidiniumbromid

### (Formel Ia: A⁻ = Br⁻)

Umesterung und Quarternisierung werden wie unter Beispiel 1 beschrieben durchgeführt.

Die entstandene Diastereomerenmischung aus (3R,2'S)- und (3R,2'R)-[(2-Cyclopentyl-2-hydroxy-2-phenylacetyl)oxy]-1,1-dimethylpyrrolidiniumbromid wird im Verhältnis von etwa eins zu eins isoliert.

3 g dieses Diastereomerengemisches werden in 21 ml Aceton, welches einen Wassergehalt von 1 % aufweist, suspendiert und 6 Stunden bei Raumtemperatur gerührt. Absaugen des Feststoffes liefert 1,047 g, worin das erwünschte Diastereomer mit 93% de enthalten ist

### Beispiel 17

### Herstellung von (3R,2'R)-[(2-Cyclopentyl-2-hydroxy-2-phenylacetyl)oxy]-1,1-dimethylpyrrolidiniumbromid

### (Formel la: A⁻ = Br⁻)

Umesterung und Quarternisierung werden wie unter Beispiel 1 beschrieben durchgeführt.

Die entstandene Diastereomerenmischung aus (3R,2'S)- und (3R,2'R)-[(2-Cyclopentyl-2-hydroxy-2-phenylacetyl)oxy]-1,1-dimethylpyrrolidiniumbromid wird im Verhältnis von etwa eins zu eins isoliert.

8 g dieses Diastereomerengemisches werden in 40 ml siedendem Isopropanol, welches einen Wassergehalt von 0,5 % aufweist, gelöst und anschließend die Lösung über einen Zeitraum von 3 Stunden auf Raumtemperatur abgekühlt. Nach weiteren 3 Stunden Rühren bei Raumtemperatur, wird das ausgefallene Produkt abgesaugt. Man erhält 2,227 g Feststoff, worin das erwünschte Diastereomer mit 96 % de enthalten ist.

## Patentansprüche

1. Verfahren zur Herstellung
a) des 3R,2'R-Stereoisomers von Glycopyrroniumbromid oder -iodid (Formel II: A = Br oder I) 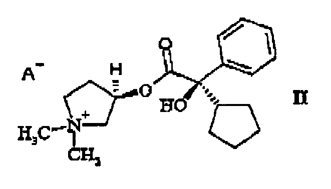 oder
b) des bezüglich der relativen Konfiguration seiner beiden Chiralitätszentren identischen 3R,2'S-Stereoisomers des Thienyl-Analogons von Glycopyrronium (Formel VI: A = Br oder I) 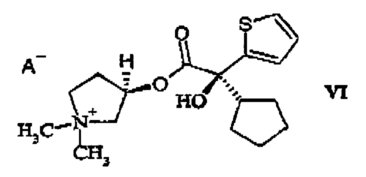
**dadurch gekennzeichnet, dass**
a) das Diastereomerengemisch bestehend aus dem 3R,2'R- und 3R,2'S-Isomer (Formel III), welches aus einem enatiomerenreinen Alkoholteil und einem razemischen Säureteil zusammengesetzt ist, 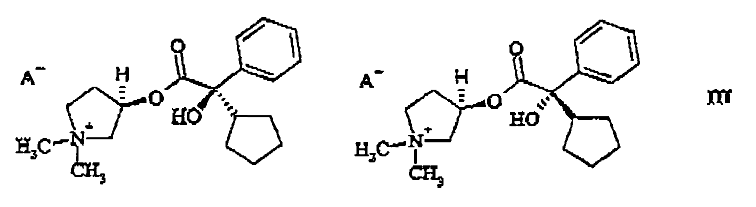 oder
b) das Diastereomerengemisch bestehend aus dem 3R,2'S- und 3R,2'R-Isomer (Formel VII), welches aus einem enatiomerenreinen Alkoholteil und einem razemischen Säureteil zusammengesetzt ist, 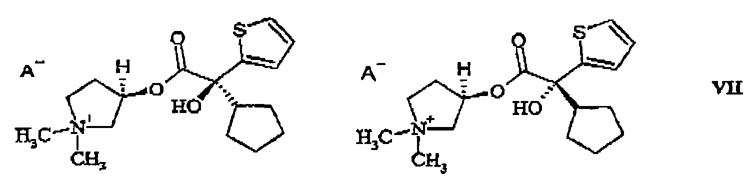
eingesetzt wird und dass die oben genannten quartären Diastereomerengemische (der Formeln III und VII) durch eine Quatemisiemung der entsprechenden tertiären, basischen Diasteremerengemische erhalten werden und dass
bei der Quaternisierung das jeweils zu isolierende, besonders gut kristallisierende Stereoisomer der Formel II bzw. der Formel VI nach der Reaktion stark angereichert als Niederschlag erhalten wird, wobei Lösungsmittel oder Lösungsmittelgemische verwendet werden, die vorzugsweise mindestens eines ausgewählt aus der Gruppe bestehend aus verzweigten und unverzweigten Alkoholen mit niederem Molekulargewicht wie Methanol, Ethanol, Isopropanol, 1-Propanol, tert-Buatnol, Isobutanol, n-Butanol, sowie Aceton, Butanon, Acetronitril enthalten
und/oder
die oben genannten quartären Diastereomerengemische (gegebenenfalls in bereits angereicherter Form) umkristallisiert werden und das jeweils zu isolierende, besonders gut kristallisierende Stereoisomer der Formel II oder VI stark angereichert als Niederschlag erhalten wird, wobei zum Lösen Methanol und/oder Ethanol verwendet wird und die Kristallisation mit Ethylacetat und/oder tert-Butylmethylether herbeigeführt wird.

2. Verfahren nach Anspruch 1 zur Herstellung
a) des 3R,2'R-Stereoisomers von Glycopyrroniumbromid oder -iodid (Formel II: A = Br oder I) 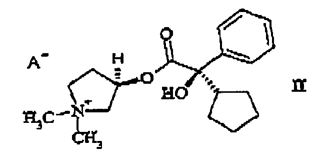 oder
b) des bezüglich der relativen Konfiguration seiner beiden Chiralitätszentren identischen 3R,2'S-Stereoisomers des Thienyl-Analogons von Glycopyrronium (Formel VI: A = Br oder I) 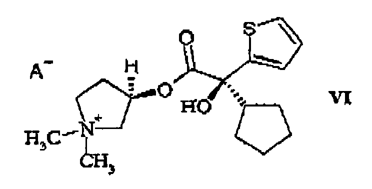
**dadurch gekennzeichnet, dass**
das Diastereomerengemisch bestehend aus dem 3R,2'R- und 3S,2'R-Isomer (Formel IIIb), welches aus einem razemischen Alkoholteil und einem enatiomerenreinen Säureteil zusammengesetzt ist, 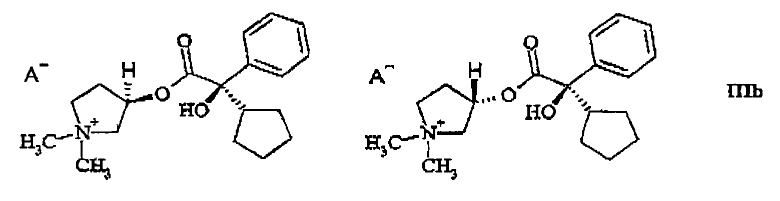 oder das Diastereomerengemisch bestehend aus dem 35,2'S- und 3R,2'S-Isomer (Formel VIIb), welches aus einem razemischen Alkoholteil und einem enatiomerenreinen Säureteil zusammengesetzt ist, 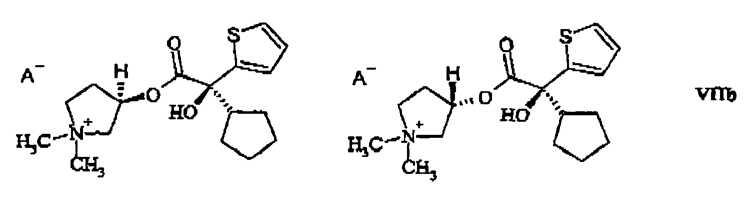 eingesetzt wird und dass die oben genannten quartären Diastereomerengemische (der Formeln IIIb und VIIb) durch eine Quatemisierung der entsprechenden tertiären, basischen Diastereomerengemische erhalten werden und dass
Bei der Quaternisierung das jeweils zu isolierende, besonders gut kristallisierende Stereoisomer der Formel II bzw. der Formel VI nach der Reaktion stark angereichert als Niederschlag erhalten wird, wobei Lösungsmittel oder Lösungsmittelgemische verwendet werden, die vorzugsweise mindestens eines ausgewählt aus der Gruppe bestehend aus verzweigten und unverzweigten Alkoholen mit niederem Molekulargewicht wie Methanol, Ethanol, Isopropanol, 1-Propanol, tert-Buatnol, Isobutanol, n-Butanol, sowie Aceton, Butanon, Acetronitril enthalten
und/oder
Die oben genannten quartären Diastereomerengemische (gegebenenfalls in bereits angereicherter Form) umkristallisiert werden und das jeweils zu isolierende, besonders gut kristallisierende Stereoisomer der Formel II bzw. der Formel VI stark angereichert als Niederschlag erhalten wird, wobei zum Lösen Methanol und/oder Ethanol verwendet wird und die Kristallisation mit Ethylacetat und/oder tert-Butylmethylether herbeigeführt wird.

3. Verfahren nach Anspruch 1 zur Herstellung der Antipoden, nämlich
a) des 3S,2'R-Isomers (Formel IV A = Br oder I) 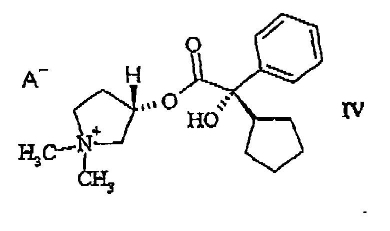 oder
b) des 3S,2'R-Isomers (Formel VIII, A = Br oder I) 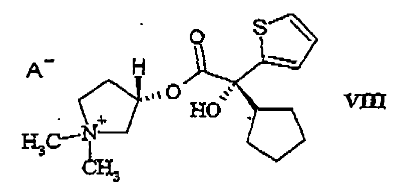
**dadurch gekennzeichnet, dass**
das Diastereomerengemisch bestehend aus dem 3S,2'R- und 3S,2'S-Isomer (Formel V) 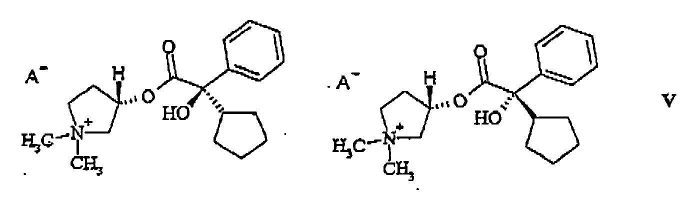 oder das Diastereomerengemisch bestehend aus dem 3S,2'S- und 3S,2'R-Isomer (Formel IX) 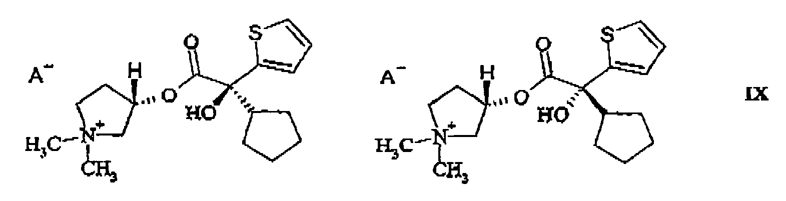 bei dem jeweils die Alkoholkomponente eine 3S-Konfiguration aufweist,
eingesetzt wird und dass die oben genannten quartären Diastereomerengemische (der Formeln V und IX) durch eine Quaternisierung der entsprechenden tertiären, basischen Diastereomerengemische erhalten werden und dass
bei der Quatemisierung das jeweils zu isolierende, besonders gut kristallisierende Stereoisomer der Formel IV bzw. der Formel VIII nach der Reaktion stark angereichert als Niederschlag erhalten wird, wobei Lösungsmittel oder Lösungsmittelgemische verwendet werden, die vorzugsweise mindestens eines ausgewählt aus der Gruppe bestehend aus verzweigten und unverzweigten Alkoholen mit niederem Molekulargewicht wie Methanol, Ethanol, Isopropanol, 1-Propanol, tert-Buatnol, Isobutanol, n-Butanol, sowie Aceton, Butanon, Acetronitril enthalten
und/oder
die oben genannten quartären Diastereomerengemische (gegebenenfalls in bereits angereicherter Form) umkristallisiert werden und das jeweils zu isolierende, besonders gut kristallisierende Stereoisomer der Formel IV bzw. der Formel VIII stark angereichert als Niederschlag erhalten wird, wobei zum Lösen Methanol und/oder Ethanol verwendet wird und die Kristallisation mit Ethylacetat und/oder tert-Butylmethylether herbeigeführt wird.

4. Verfahren nach Ansprüchen 1 und 2 zur Herstellung der Antipoden, nämlich
a) des 3S,2'S-Isomers (Formel IV, A = BR oder I) 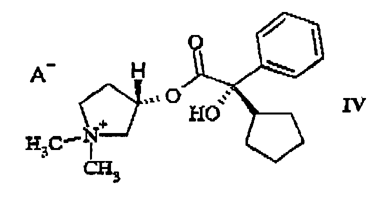 oder
b) des 3S,2'R-Isomers (Formel VIII, A = BR oder I) 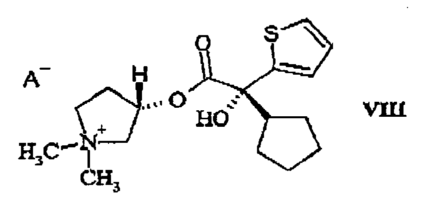
**dadurch gekennzeichnet, dass**
das Diasteromerengemisch bestehend aus dem 3R,2'S- und 3S,2'S-Isomer (Formel Vb) 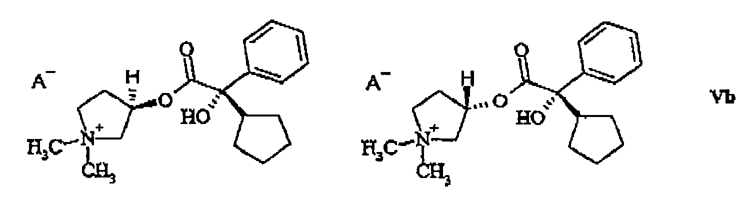 oder
das Diastereomerengemisch bestehend aus dem 3R,2'R- und 3S,2'R-Isomer (Formel IXb) 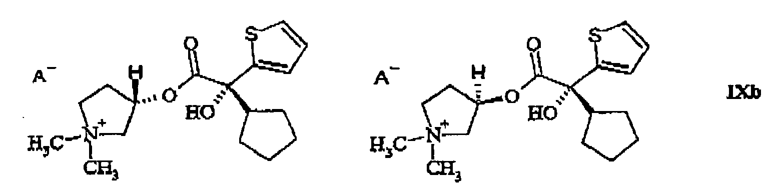 bei dem die Säurekomponente im Falle von Vb der 2'S-Konfiguration bzw. Im Falle von IXb die in der räumlichen Anordnung entsprechende 2'R-Konfiguration aufweist,
eingesetzt wird und dass die oben genannten quartären Diastereomerengemische (der Formeln Vb und IXb) durch die Quaternisierung der entsprechenden tertiären, basischen Diastereomerengemische erhalten werden und dass
bei der Quatemisierung das jeweils zu isolierende, besonders gut kristallisierende Stereoisomer der Formel IV bzw. Der Formel VIII nach der Reaktion stark angereichert als Niederschlag erhalten wird, wobei Lösungsmittel oder Lösungsmittelgemische verwendet werden, die vorzugsweise mindestens eines ausgewählt aus der Gruppe bestehend aus verzweigten und unverzweigten Alkoholen mit niederem Molekulargewicht wie Methanol, Ethanol, Isopropanol, 1-Propanol, tert-Buatnol, Isobutanol, n-Butanol, sowie Aceton, Butanon, Acetronitril enthalten
und/oder
die oben genannten quartären Diastereomerengemische (Gegebenenfalls in bereits abgereicherter Form) umkristallisiert werden und das jeweils zu isolierende, besonders gut kristallisierende Stereoisomer der Formel IV bzw. Der Formel VII stark angereichert als Niederschlag erhalten wird, wobei zum Lösen Methanol und/oder Ethanol verwendet wird und die Kristallisation mit Ethylacetat und/oder tert-Butylmethylether herbeigeführt wird.

5. Verfahren nach einem der Ansprüche 1 bis 4, **dadurch gekennzeichnet, dass** bevorzugt Lösungsmittel mit einem Wassergehalt verwendet werden und dass nur das besonders gut kristallisierende Diastereomer als Feststoff anfällt, während das andere Diastereomer in Lösung bleibt oder als Öl anfällt.

6. Verfahren nach einem der Ansprüche 1 bis 5, **dadurch gekennzeichnet, dass** bei der Quaternisierung zu den genannten Diastereomerengemischen der quartären Salze ein Lösungsmittel wie z.B. Isopropanol oder Aceton verwendet und das besonders gut kristallisierende Stereoisomer im entstehenden Niederschlag in stark angereicherter Form isoliert wird.

7. Verfahren nach einem der Ansprüche 1 bis 6, bei dem zum Umkristallisieren das Diastereomerengemisch in einem erhitzten Lösungsmittel gelöst wird und durch Abkühlung Kristallisation erfolgt.

8. Verfahren nach Anspruch 7, bei dem das Diastereomerengemsich in der Siedehitze in 2-Propanol oder Ethanol gelöst wird und die Kristallisation bei Abkühlung auf Raumtemperatur oder darunter erfolgt.

9. Verfahren nach einem der Ansprüche 1 bis 8 als Vorreinigungsstufe zum Erzielen einer primären Diastereomerenanreicherung oder, bei bereits erfolgter Anreicherung, zu einer weiteren Steigerung der Diastereomerenreinheit.

10. Verfahren nach einem der Ansprüche 1 bis 9, **dadurch gekennzeichnet, dass** der Niederschlag des gewünschten Stereoisomers bei der Quatemisierung der tertiären Basen zu den genannten Diastereomerengemischen der Ammoniumsalze und/oder beim Umkristallisieren der quartären Salze zur Entfernung des unerwünschten Isomers nachgewaschen bzw. digeriert wird.

11. Verfahren nach einem der Ansprüche 1 bis 10, **dadurch gekennzeichnet, dass** bei der Quatemisierung ein Lösungsmittel mit einem Wassergehalt von vorzugsweise weniger als 5 % verwendet wird.

12. Verfahren nach einem der Ansprüche 1 bis 11, **dadurch gekennzeichnet, dass** bei der Quatemisierung ein Lösungsmittel mit einem Wassergehalt von etwa 0,5 - 2 % verwendet wird.

13. Verfahren nach einem der Ansprüche 1 bis 12, **dadurch gekennzeichnet, dass** bei der Quaternisierung ein Lösungsmittel mit einem Wassergehalt von etwa 1 % verwendet wird.

14. Verfahren nach einem der Ansprüche 1 bis 13, **dadurch gekennzeichnet, dass** beim Umkristallisieren ein Lösungsmittel mit einem Wassergehalt von vorzugsweise etwa 0,2 - 3 % verwendet wird.

15. Verfahren nach einem der Ansprüche 1 bis 14, **dadurch gekennzeichnet, dass** beim Umkristallisieren ein Lösungsmittel mit einem Wassergehalt von etwa 0,5 % verwendet wird.

16. Verfahren nach einem der Ansprüche 1 bis 15, bevorzugt zur Anreicherung des 3R,2'R-Isomers von Glycopyrroniumbromid.

## Claims

1. Method for the production
a) of the 3R,2'R stereoisomer of glycopyrronium bromide or iodide (formula II: A = Br or I) 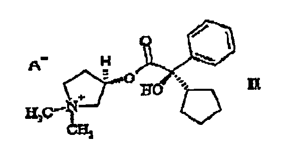 or
b) of the 3R,2'S stereoisomer of the thienyl analogon of glycopyrronium, which is identical with regard to the relative configuration of its two chirality centres (formula VI: A = Br or I) 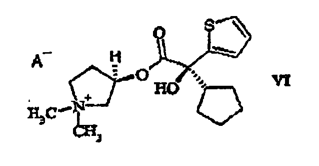
**characterized in that**
the diastereomer mixture consisting of the 3R,2'R and 3R,2'S isomer (formula III), which is composed of an enantiomerically pure alcohol part and a racemic acid part, 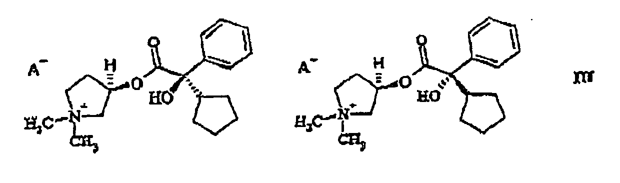 or
the diastereomer mixture consisting of the 3R,2'S and 3R,2'R isomer (formula VII), which is composed of an enantiomerically pure alcohol part and a racemic acid part, 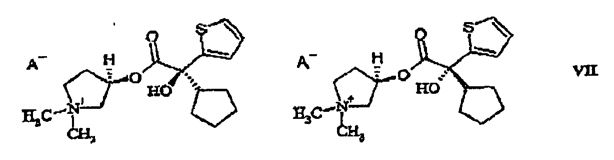 is used and that the aforementioned quaternary diastereomer mixtures (of the formulae III and VII) are obtained by a quaternization of the corresponding tertiary, basic diastereomer mixtures and that during the quaternization, the particularly readily crystallizing stereoisomer of the formula II or of the formula VI to be isolated in each case is obtained after the reaction in highly enriched form as a precipitate, where solvents or solvent mixtures are used which preferably comprise at least one selected from the group consisting of branched and unbranched alcohols with a low molecular weight, such as methanol, ethanol, isopropanol, 1-propanol, tert-butanol, isobutanol, n-butanol, and also acetone, butanone, acetonitrile,
and/or
the aforementioned quaternary diastereomer mixtures (optionally in already enriched form) are recrystallized and the particularly readily crystallizing stereoisomer of the formula II or VI to be isolated in each case is obtained in highly enriched form as a precipitate, where, for the purposes of dissolution, methanol and/or ethanol is used and the crystallization is carried out with ethyl acetate and/or tert-butyl methyl ether.

2. Method according to Claim 1 for the production
a) of the 3R,2'R stereoisomer of glycopyrronium bromide or iodide (formula II: A = Br or I) 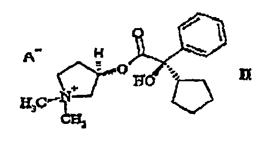 or
b) of the 3R,2'S stereoisomer of the thienyl analogon of glycopyrronium, which is identical with regard to the relative configuration of its two chirality centres (formula VI: A = Br or I) 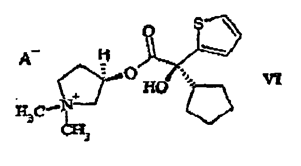
**characterized in that**
the diastereomer mixture consisting of the 3R,2'R and 3S,2'R isomer (formula IIIb), which is composed of an racemic alcohol part and an enantiomerically pure acid part, 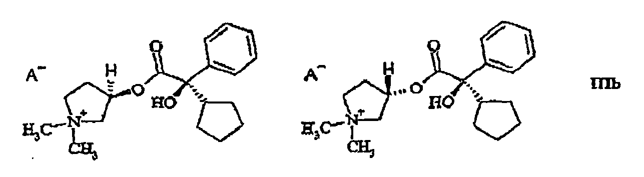 or the diastereomer mixture consisting of the 35,2'S and 3R,2'S isomer (formula VIIb), which is composed of a racemic alcohol part and an enantiomerically pure acid part, 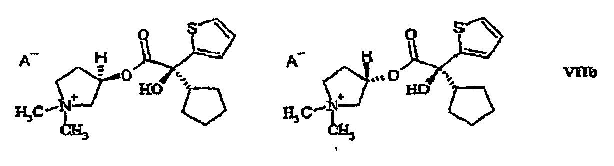 is used and that the aforementioned quaternary diastereomer mixtures (of the formulae IIIb and VIIb) are obtained by a quaternization of the corresponding tertiary, basic diastereomer mixtures and that
during the quaternization, the particularly readily crystallizing stereoisomer of the formula II or of the formula VI to be isolated in each case is obtained after the reaction in a highly enriched form as a precipitate, where solvents or solvent mixtures are used which preferably comprise at least one selected from the group consisting of branched and unbranched alcohols with a low molecular weight such as methanol, ethanol, isopropanol, 1-propanol, tert-butanol, isobutanol, n-butanol, and also acetone, butanone, acetonitrile
and/or
the aforementioned quaternary diastereomer mixtures (optionally in already enriched form) are recrystallized and the particularly readily crystallizing stereoisomer of the formula II or of the formula VI to be isolated in each case is obtained in highly enriched form as precipitate, where, for the purposes of the dissolution, methanol and/or ethanol is used and the crystallization is carried out with ethyl acetate and/or tert-butyl methyl ether.

3. Method according to Claim 1 for the production of antipodes, namely
a) of the 3S,2'R isomer (formula IV A = Br or I) 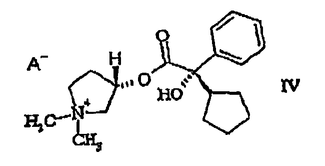 or
b) of the 3S,2'R isomer (formula VIII, A = Br or I) 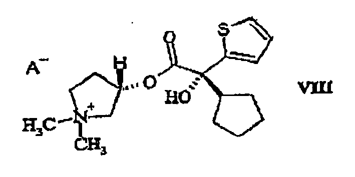
**characterized in that**
the diastereomer mixture consisting of the 3S,2'R and 3S, 2'S isomer (formula V) 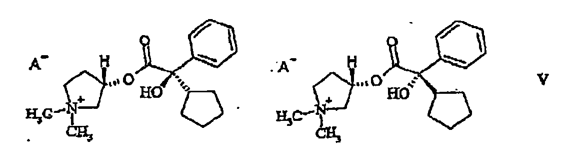 or the diastereomer mixture consisting of the 3S,2'S and 3S,2'R isomer (formula IX) 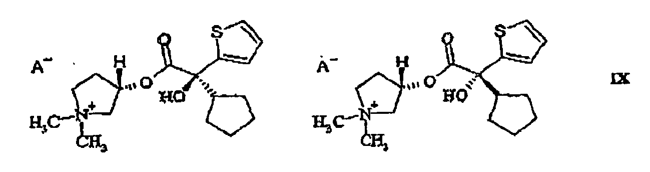 in which in each case the alcohol component has a 3S configuration,
is used and that the aforementioned quaternary diastereomer mixtures (of the formulae V and IX) are obtained by a quaternization of the corresponding tertiary, basic diastereomer mixtures and that
during the quaternization, the particularly readily crystallizing stereoisomer of the formula IV or of the formula VIII to be isolated in each case is obtained after the reaction in highly enriched form as a precipitate, where solvents or solvent mixtures are used which preferably comprise at least one selected from the group consisting of branched and unbranched alcohols with a low molecular weight such as methanol, ethanol, isopropanol, 1-propanol, tert-butanol, isobutanol, n-butanol, and also acetone, butanone, acetonitrile
and/or
the aforementioned quaternary diastereomer mixtures (optionally in already enriched form) are recrystallized and the particularly readily crystallizing stereoisomer of the formula IV or of the formula VIII to be isolated in each case is obtained in highly enriched form as a precipitate, where, for the purposes of the dissolution, methanol and/or ethanol is used and the crystallization is carried out with ethyl acetate and/or tert-butyl methyl ether.

4. Method according to Claims 1 and 2 for the production of the antipodes, namely
a) of the 3S,2'S isomer (formula IV, A = Br or I) 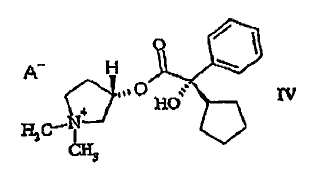 or
b) of the 3S,2'R isomer (formula VIII, A = Br or I) 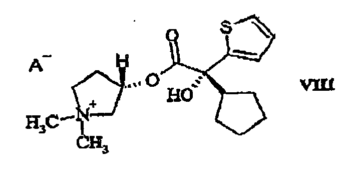
**characterized in that**
the diastereomer mixture consisting of the 3R,2'S and 3S,2'S isomer (formula Vb) 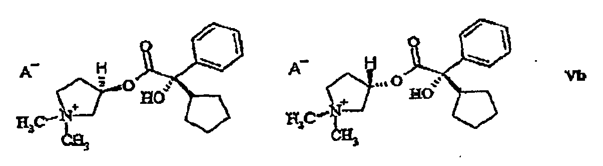 or
the diastereomer mixture consisting of the 3R,2'R and 3S,2'R isomer (formula IXb) 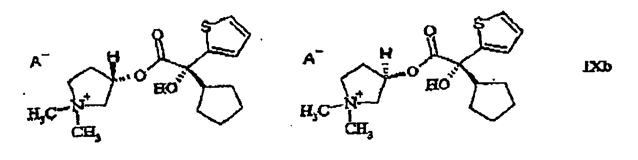 in which the acid component in the case of Vb has the 2'S configuration and in the case of IXb the 2'R configuration corresponding in the spatial arrangement, is used and that the aforementioned quaternary diastereomer mixtures (of the formulae Vb and IXb) are obtained by the quaternization of the corresponding tertiary, basic diastereomer mixtures and that
during the quaternization, the particularly readily crystallizing stereoisomer of the formula IV or of the formula VIII to be isolated in each case is obtained after the reaction in highly enriched form as a precipitate, where solvents or solvent mixtures are used which preferably comprise at least one selected from the group consisting of branched and unbranched alcohols with a low molecular weight such as methanol, ethanol, isopropanol, 1-propanol, tert-butanol, isobutanol, n-butanol, and also acetone, butanone, acetonitrile
and/or
the aforementioned quaternary diastereomer mixtures (optionally in already enriched form) are recrystallized and the particularly readily crystallizing stereoisomer of the formula IV or of the formula VIII to be isolated in each case is obtained in highly enriched form as a precipitate, where, for the purposes of the dissolution, methanol and/or ethanol is used and the crystallization is carried out with ethyl acetate and/or tert-butyl methyl ether.

5. Method according to one of Claims 1 to 4, **characterized in that** preferably solvents with a water content are used and that only the particularly readily crystallizing diastereomer is produced as solid while the other diastereomer remains in solution or is produced as oil.

6. Method according to one of Claims 1 to 5, **characterized in that**, during the quaternization to give said diastereomer mixtures of the quaternary salts, a solvent such as e.g. isopropanol or acetone is used and the particularly readily crystallizing stereoisomer is isolated in the resulting precipitate in highly enriched form.

7. Method according to one of Claims 1 to 6, in which, for the recrystallization, the diastereomer mixture is dissolved in a heated solvent and crystallization takes place by means of cooling.

8. Method according to Claim 7, in which the diastereomer mixture is dissolved at the boiling temperature in 2-propanol or ethanol and the crystallization takes place upon cooling to room temperature or below.

9. Method according to one of Claims 1 to 8 as prepurification stage for achieving a primary diastereomer enrichment or, in the event of enrichment that has already taken place, for the purposes of further increasing the diastereomer purity.

10. Method according to one of Claims 1 to 9, **characterized in that** the precipitate of the desired stereoisomer is after-washed and/or digested during the quaternization of the tertiary bases to give the specified diastereomer mixtures of the ammonium salts and/or during the recrystallization of the quaternary salts for the purposes of removing the undesired isomer.

11. Method according to one of Claims 1 to 10, **characterized in that**, during the quaternization, a solvent with a water content of preferably less than 5% is used.

12. Method according to one of Claims 1 to 11, **characterized in that**, during the quaternization, a solvent with a water content of about 0.5-2% is used.

13. Method according to one of Claims 1 to 12, **characterized in that**, during the quaternization, a solvent with a water content of about 1% is used.

14. Method according to one of Claims 1 to 13, **characterized in that**, during the recrystallization, a solvent with a water content of preferably about 0.2-3% is used.

15. Method according to one of Claims 1 to 14, **characterized in that**, during the recrystallization, a solvent with a water content of about 0.5% is used.

16. Method according to one of Claims 1 to 15, preferably for enriching the 3R,2'R isomer of glycopyrronium bromide.

## Revendications

1. Procédé pour la préparation
a) du stéréoisomère 3R,2'R de bromure ou d'iodure de glycopyrronium (formule II : A = Br ou I) 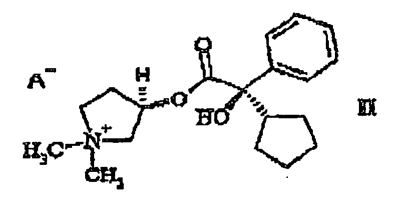 ou
b) du stéréoisomère 3R,2'S de l'analogue thiényle du glycopyrronium, identique quant à la configuration relative de ses deux centres de chiralité (formule VI : A = Br ou I) 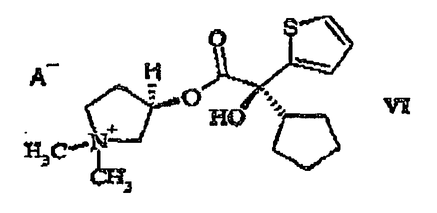
**caractérisé en ce qu'**on utilise
a) le mélange de diastéréoisomères constitué de l'isomère 3R,2'R et de l'isomère 3R,2'S (formule III), qui est composé d'un fragment alcool sous forme d'énantiomère pur et d'un fragment acide racémique 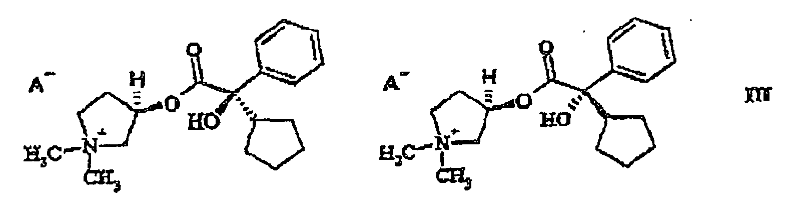 ou
b) le mélange de diastéréoisomères constitué de l'isomère 3R,2'S et de l'isomère 3R,2'R (formule VII), qui est composé d'un fragment alcool sous forme d'énantiomère pur et d'un fragment acide racémique, 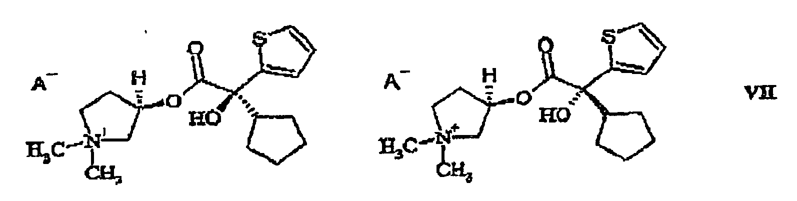
et **en ce qu'**on obtient les mélanges de diastéréoisomères quaternaires (de formules III et VII) indiqués ci-dessus par une quaternisation des mélanges de diastéréoisomères basiques tertiaires correspondants et **en ce que**
dans la quaternisation on obtient en tant que précipité, fortement enrichi après la réaction, le stéréoisomère de formule II ou, respectivement, de formule VI, particulièrement bien cristallisable, respectivement à isoler, en utilisant des solvants ou mélanges de solvants qui de préférence contiennent au moins un solvant choisi dans le groupe consistant en des alcools ramifiés et des alcools non ramifiés, de faible masse moléculaire, tels que le méthanol, l'éthanol, l'isopropanol, le 1-propanol, le tert-butanol, l'isobutanol, le n-butanol, ainsi que l'acétone, la butanone, l'acétonitrile
et/ou
on fait recristalliser les mélanges de diastéréoisomères quaternaires nommés ci-dessus (éventuellement sous forme déjà enrichie) et on obtient en tant que précipité, fortement enrichi, le stéréoisomère de formule II ou VI particulièrement bien cristallisable, respectivement à isoler, en utilisant du méthanol et/ou de l'éthanol pour la dissolution et en provoquant la cristallisation avec de l'acétate d'éthyle et/ou de l'oxyde de méthyle et de tert-butyle.

2. Procédé selon la revendication 1 pour la préparation
a) du stéréoisomère 3R,2'R de bromure ou d'iodure de glycopyrronium (formule II : A = Br ou I) 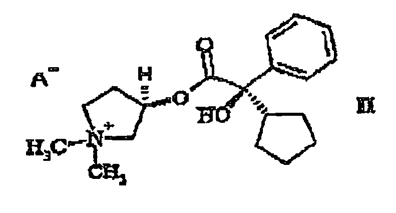 ou
b) du stéréoisomère 3R,2'S de l'analogue thiényle de glycopyrronium, identique quant à la configuration relative de ses deux centres de chiralité (formule VI : A = Br ou I) 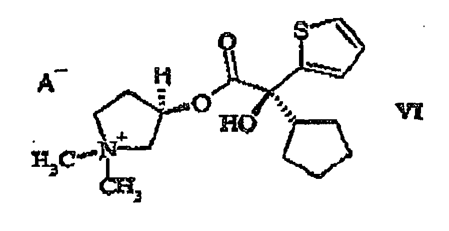
**caractérisé en ce qu'**on utilise
le mélange de diastéréoisomères constitué de l'isomère 3R,2'R et de l'isomère 3S,2'R (formule IIIb), qui est composé d'un fragment alcool racémique et d'un fragment acide sous forme d'énantiomère pur, 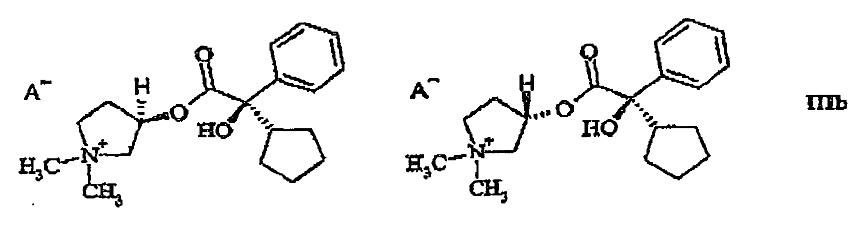 ou le mélange de diastéréoisomères constitué de l'isomère 3S,2'S et de l'isomère 3R,2'S (formule VIIb), qui est composé d'un fragment alcool racémique et d'un fragment acide sous forme d'énantiomère pur, 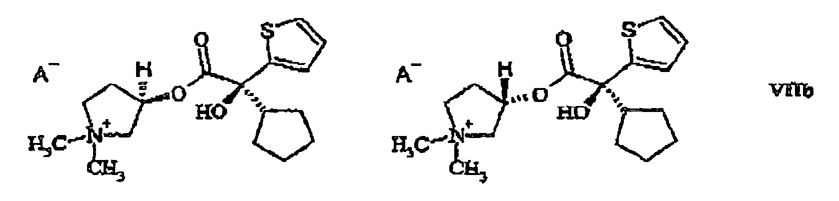 et **en ce qu'**on obtient les mélanges de diastéréoisomères quaternaires (de formules IIIb et VIIb) indiqués ci-dessus par une quaternisation des mélanges de diastéréoisomères basiques tertiaires correspondants et **en ce que**
dans la quaternisation on obtient en tant que précipité, fortement enrichi après la réaction, le stéréoisomère de formule II ou, respectivement, de formule VI, particulièrement bien cristallisable, respectivement à isoler, en utilisant des solvants ou mélanges de solvants qui de préférence contiennent au moins un solvant choisi dans le groupe consistant en des alcools ramifiés et des alcools non ramifiés, de faible masse moléculaire, tels que le méthanol, l'éthanol, l'isopropanol, le 1-propanol, le tert-butanol, l'isobutanol, le n-butanol, ainsi que l'acétone, la butanone, l'acétonitrile
et/ou
on fait recristalliser les mélanges de diastéréoisomères quaternaires nommés ci-dessus (éventuellement sous forme déjà enrichie) et on obtient en tant que précipité, fortement enrichi, le stéréoisomère de formule II ou, respectivement, de formule VI, particulièrement bien cristallisable, respectivement à isoler, en utilisant du méthanol et/ou de l'éthanol pour la dissolution et en provoquant la cristallisation avec de l'acétate d'éthyle et/ou de l'oxyde de méthyle et de tert-butyle.

3. Procédé selon la revendication 1 pour la préparation des antipodes, à savoir
a) de l'isomère 3S,2'R (formule IV : A = Br ou I) 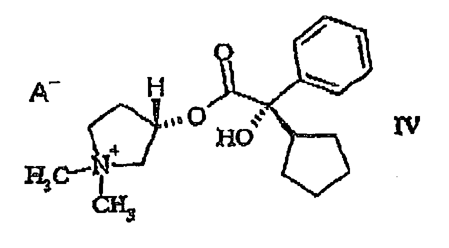 ou
b) de l'isomère 3S,2'R (formule VIII : A = Br ou I) 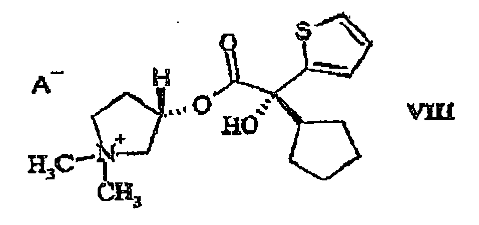
**caractérisé en ce qu'**on utilise
le mélange de diastéréoisomères constitué de l'isomère 3S,2'R et de l'isomère 3S,2'S (formule V) 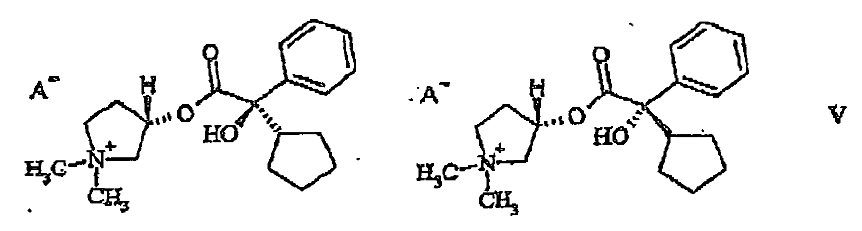 ou le mélange de diastéréoisomères constitué de l'isomère 3S,2'S et de l'isomère 3S,2'R (formule IX), 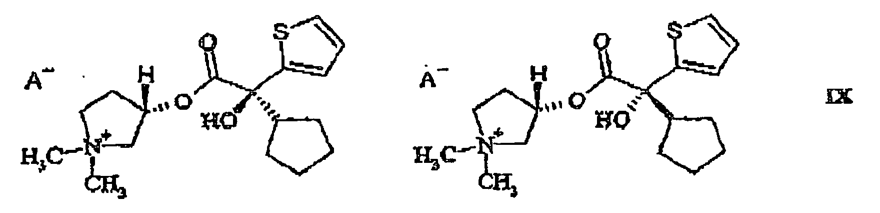 dans lequel le composant alcool présente chaque fois une configuration 3S,
et **en ce qu'**on obtient les mélanges de diastéréoisomères quaternaires (de formules V et IX) indiqués ci-dessus par une quaternisation des mélanges de diastéréoisomères basiques tertiaires correspondants et **en ce que**
dans la quaternisation on obtient en tant que précipité, fortement enrichi après la réaction, le stéréoisomère de formule IV ou, respectivement, de formule VIII, particulièrement bien cristallisable, respectivement à isoler, en utilisant des solvants ou mélanges de solvants qui de préférence contiennent au moins un solvant choisi dans le groupe consistant en des alcools ramifiés et des alcools non ramifiés, de faible masse moléculaire, tels que le méthanol, l'éthanol, l'isopropanol, le 1-propanol, le tert-butanol, l'isobutanol, le n-butanol, ainsi que l'acétone, la butanone, l'acétonitrile
et/ou
on fait recristalliser les mélanges de diastéréoisomères quaternaires nommés ci-dessus (éventuellement sous forme déjà enrichie) et on obtient en tant que précipité, fortement enrichi, le stéréoisomère de formule IV ou, respectivement, de formule VIII, particulièrement bien cristallisable, respectivement à isoler, en utilisant du méthanol et/ou de l'éthanol pour la dissolution et en provoquant la cristallisation avec de l'acétate d'éthyle et/ou de l'oxyde de méthyle et de tert-butyle.

4. Procédé selon les revendications 1 et 2 pour la préparation des antipodes, à savoir
a) de l'isomère 3S,2'S (formule IV, A = Br ou I) 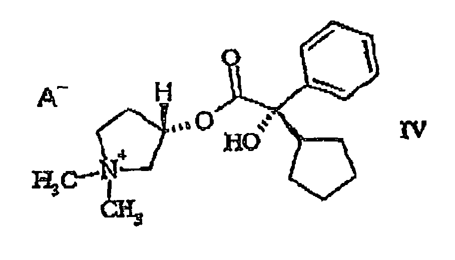 ou
b) de l'isomère 3S,2'R (formule VIII, A = Br ou I) 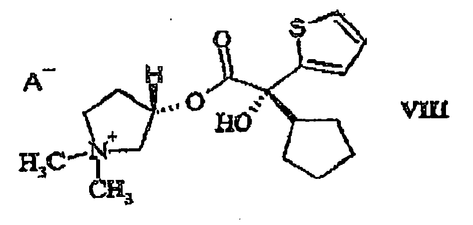
**caractérisé en ce qu'**on utilise
le mélange de diastéréoisomères constitué de l'isomère 3R,2'S et de l'isomère 3S,2'S (formule Vb) 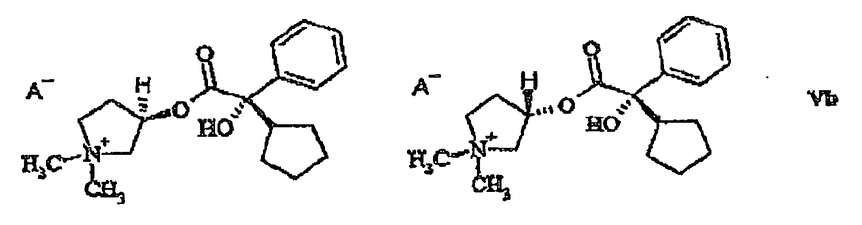 ou le mélange de diastéréoisomères constitué de l'isomère 3R,2'R et de l'isomère 3S,2'R (formule IXb) 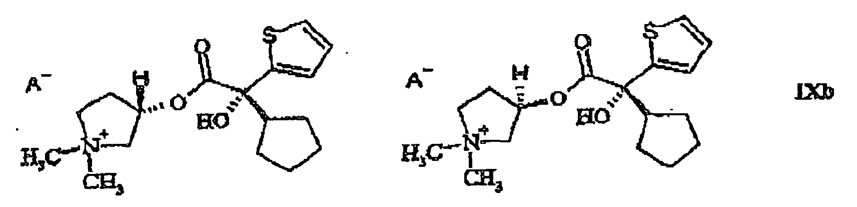 dans lequel le composant alcool dans le cas de Vb présente la configuration 2'S ou, respectivement, dans le cas de IXb présente la configuration 2'R correspondante dans la disposition spatiale,
et **en ce qu'**on obtient les mélanges de diastéréoisomères quaternaires (de formules Vb et IXb) indiqués ci-dessus par la quaternisation des mélanges de diastéréoisomères basiques tertiaires correspondants et **en ce que**
dans la quaternisation on obtient en tant que précipité, fortement enrichi après la réaction, le stéréoisomère de formule IV ou, respectivement, de formule VIII, particulièrement bien cristallisable, respectivement à isoler, en utilisant des solvants ou mélanges de solvants qui de préférence contiennent au moins un solvant choisi dans le groupe consistant en des alcools ramifiés et des alcools non ramifiés, de faible masse moléculaire, tels que le méthanol, l'éthanol, l'isopropanol, le 1-propanol, le tert-butanol, l'isobutanol, le n-butanol, ainsi que l'acétone, la butanone, l'acétonitrile
et/ou
on fait recristalliser les mélanges de diastéréoisomères quaternaires nommés ci-dessus (éventuellement sous forme déjà enrichie) et on obtient en tant que précipité, fortement enrichi, le stéréoisomère de formule IV ou, respectivement, de formule VIII particulièrement, bien cristallisable, respectivement à isoler, en utilisant du méthanol et/ou de l'éthanol pour la dissolution et en provoquant la cristallisation avec de l'acétate d'éthyle et/ou de l'oxyde de méthyle et de tert-butyle.

5. Procédé selon l'une quelconque des revendications 1 à 4, **caractérisé en ce que** de préférence on utilise des solvants ayant une teneur en eau et **en ce que** seul précipite sous forme de solide le diastéréoisomère particulièrement bien cristallisable, tandis que l'autre diastéréoisomère reste en solution ou s'accumule sous forme d'huile.

6. Procédé selon l'une quelconque des revendications 1 à 5, **caractérisé en ce que** dans la quaternisation conduisant aux mélanges de diastéréoisomères nommés des sels quaternaires on utilise un solvant comme par exemple l'isopropanol ou l'acétone et dans le précipité résultant on isole sous forme fortement enrichie le stéréoisomère particulièrement bien cristallisable.

7. Procédé selon l'une quelconque des revendications 1 à 6, dans lequel pour la recristallisation on dissout le mélanges de diastéréoisomères dans un solvant chauffé et on effectue la cristallisation par refroidissement.

8. Procédé selon la revendication 7, dans lequel on dissout le mélange de diastéréoisomères à l'ébulllition dans du 2-propanol ou de l'éthanol et la cristallisation s'effectue lors du refroidissement jusqu'à la température ou au-dessous.

9. Procédé selon l'une quelconque des revendications 1 à 8, en tant qu'étape de prépurification pour parvenir à un enrichissement primaire en diastéréoisomères, ou, dans le cas d'enrichissement déjà effectué, à une nouvelle augmentation de la pureté des diastéréosiomères.

10. Procédé selon l'une quelconque des revendications 1 à 9, **caractérisé en ce que** lors de la quaternisation des bases tertiaires conduisant aux mélanges nommés de diastéréoisomères des sels d'ammonium et/ou lors de la recristallisation des sels quaternaires, on fait digérer ou on soumet à un post-lavage le précipité du stéréoisomère recherché, afin d'éliminer l'isomère indésirable.

11. Procédé selon l'une quelconque des revendications 1 à 10, **caractérisé en ce que** dans la quaternisation on utilise un solvant ayant une teneur en eau de préférence inférieure à 5 %.

12. Procédé selon l'une quelconque des revendications 1 à 11, **caractérisé en ce que** dans la quaternisation on utilise un solvant ayant une teneur en eau d'environ 0,5 - 2 %.

13. Procédé selon l'une quelconque des revendications 1 à 12, **caractérisé en ce que** dans la quaternisation on utilise un solvant ayant une teneur en eau d'environ 1 %.

14. Procédé selon l'une quelconque des revendications 1 à 13, **caractérisé en ce que** dans la recristallisation on utilise un solvant ayant une teneur en eau de préférence d'environ 0,2 - 3 %.

15. Procédé selon l'une quelconque des revendications 1 à 14, **caractérisé en ce que** dans la recristallisation on utilise un solvant ayant une teneur en eau d'environ 0,5 %.

16. Procédé selon l'une quelconque des revendications 1 à 15, de préférence pour l'enrichissement de l'isomère 3R,2'R de bromure de glycopyrronium.
